(19) Europäisches Patentamt
European Patent Office
Office européen des brevets

(11) Veröffentlichungsnummer: **0 012 435**
**A2**

(12) # EUROPÄISCHE PATENTANMELDUNG

(21) Anmeldenummer: **79105143.6**

(22) Anmeldetag: **13.12.79**

(51) Int. Cl.³: **C 07 D 261/18**, C 07 D 413/12, A 61 K 31/42, A 61 K 31/44, C 07 C 103/58, C 07 C 103/60, C 07 D 213/30, C 07 D 213/75

(30) Priorität: **16.12.78 DE 2854438**

(43) Veröffentlichungstag der Anmeldung: **25.06.80 Patentblatt 80/13**

(84) Benannte Vertragsstaaten: **AT BE CH DE FR GB IT LU NL SE**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Kämmerer, Friedrich-Johannes, Dr., Am Weiher 27, D-6203 Hochheim am Main (DE)**
Erfinder: **Schleyerbach, Rudolf, Dr., Finkenweg 10, D-6238 Hofheim am Taunus (DE)**

(54) **Isoxazolderivate, Verfahren zu ihrer Herstellung, diese Verbindungen enthaltende Arzneimittel und bei dem Verfahren benötigte Zwischenprodukte.**

(57) Isoxazolderivate und Verfahren zu ihrer Herstellung werden beschrieben. Die Verbindungen wirken antirheumatisch, antiphlogistisch, analgetisch und antipyretisch und können als Arzneimittel verwendet werden.

EP 0 012 435 A2

0012435
HOECHST AKTIENGESELLSCHAFT HOE 78/F 274 Dr.KM/a

Isoxazolderivate, Verfahren zu ihrer Herstellung, diese Verbindungen enthaltende Mittel und ihre Verwendung

BEZEICHNUNG GEÄNDERT
siehe Titelseite

Nach DE-OS 25 24 959 und DE-OS 26 55 009 sind 5-Methylisoxazol-4-carbonsäureanilide und -carbonsäureamide heterocyclischer Amine mit antiphlogistischen und analgetischen Eigenschaften bekannt.

Gegenstand der Erfindung sind Isoxazol-4-carbonsäureamide der allgemeinen Formel I

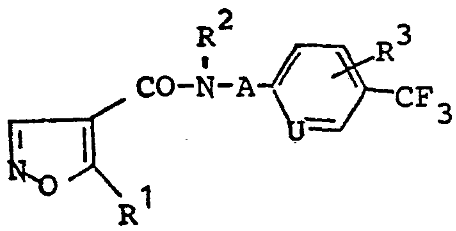

(I),

worin

$R^1$ Wasserstoff, Alkyl oder Halogenalkyl mit jeweils bis zu 4 C-Atomen,

$R^2$ Wasserstoff oder $(C_1-C_4)$Alkyl,

$R^3$ Wasserstoff oder Halogen,

U die -CH-Gruppe oder Stickstoff und

A den Rest der Formel

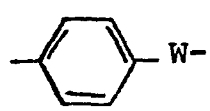

in der W für Sauerstoff oder die Carbonylgruppe steht, oder eine Einfachbindung, in welchem Fall jedoch $R^1$ dann verschieden von Methyl ist, wenn gleichzeitig $R^2$ und $R^3$ Wasserstoff und U die CH-Gruppe darstellen, bedeuten.

In $R^1$ und $R^2$ kommt als Alkyl Methyl, Äthyl, n-Propyl, i-Propyl sowie n-, i- und t-Butyl, in $R^1$ als Halogenalkyl insbesondere $-CF_3$, $-CH_2-CF_3$, $-CH_2-CHF_2$, $-CClF_2$, $-CCl_3$ in Betracht.

Bevorzugt sind unter diesen Verbindungen solche der Formel I, worin

$R^1$ und $R^2$ gleich oder verschieden sein können und Wasserstoff, Methyl oder Äthyl,

$R^3$ Wasserstoff,

U die CH-Gruppe und

A den Rest der Formel 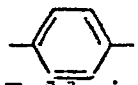 O- oder eine Einfachbindung, in welchem Fall jedoch $R^1$ verschieden von Methyl ist, wenn gleichzeitig $R^2$ Wasserstoff bedeutet, darstellen.

Bevorzugt sind weiterhin Verbindungen der Formel I, worin

$R^1$ die $CF_3$-Gruppe,

$R^2$ Wasserstoff, Methyl oder Äthyl,

$R^3$ Wasserstoff,

U die CH-Gruppe und

A eine Einfachbindung oder den Rest der Formel 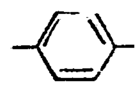 O- bedeuten.

Gegenstand der Erfindung ist auch ein Verfahren zur Herstellung von Verbindungen der Formel I, das dadurch gekennzeichnet ist, daß man

a) ein Isoxazol-4-carbonsäure-Derivat der Formel II

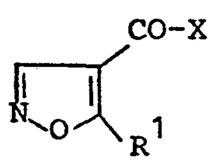

(II)

in der X entweder ein Halogenatom, vorzugsweise Chlor oder Brom, eine YO- oder ZO-CO-O-Gruppe bedeutet, wobei Y für gegebenenfalls durch Fluor, Chlor, Brom, Jod, Methyl, Äthyl, Methoxy, Äthoxy, Trifluormethyl, Nitro oder Cyan einfach, zweifach oder dreifach substituiertes Phenyl oder für den Acylrest entsprechend der Formel II (Formel II ohne X) und Z für $(C_1-C_4)$ Alkyl, Phenyl oder Benzyl stehen, und $R^1$ Wasserstoff, Alkyl oder Halogenalkyl mit jeweils bis zu 4 C-Atomen bedeutet, mit einem Amin der Formel III oder dessen Salzen,

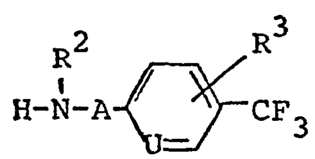

(III),

in der $R^2$, $R^3$, U und A die vorgenannten Bedeutungen haben, umsetzt oder

b) ein Acylessigsäureamid der Formel IV

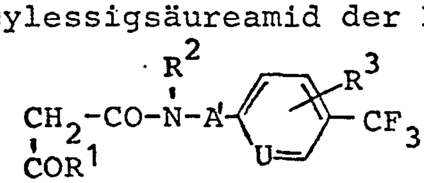

(IV),

in der

$R^1$ Alkyl oder Halogenalkyl mit jeweils bis zu 4 C-Atomen,

$R^2$ Wasserstoff oder $(C_1-C_4)$-Alkyl,

$R^3$ Wasserstoff oder Halogen,

U die CH-Gruppe oder Stickstoff und

A' den Rest der Formel 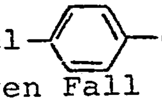O- oder eine Einfachbindung, in welchem letzteren Fall einerseits $R^1$ dann verschieden von Methyl ist, wenn gleichzeitig $R^2$ und $R^3$ Wasserstoff und U die CH-Gruppe darstellen, oder andererseits nicht gleichzeitig $R^2$ Wasserstoff und U Stickstoff sind, bedeuten,

mit einem Orthoameisensäureester der Formel V

$$HC(OR)_3 \qquad (V),$$

worin R eine $(C_1-C_4)$-Alkylgruppe, vorzugsweise Methyl oder Äthyl, darstellt,
zu 2-Alkoxymethylenacylessigsäureamid der Formel VI

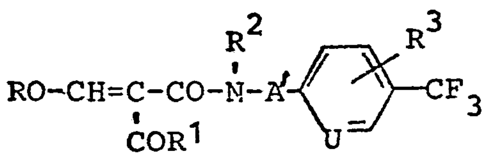
(VI)

in der die Substituenten die vorstehend angegebenen Bedeutungen haben, umsetzt und dieses anschließend mit Hydroxylamin unter Ringschluß kondensiert.

Die Reaktion nach Verfahrensvariante a) wird zweckmäßig in einem Verteilungs- oder Lösungsmittel durchgeführt, das sich unter den Reaktionsbedingungen gegenüber den Reaktionspartnern indifferent verhält. Hierfür kommen beispielsweise Nitrile, wie Acetonitril, Äther, wie Diäthyläther, Tetrahydrofuran oder Dioxan, Alkohole, wie Methanol, Äthanol, Propanol oder Isopropanol und Wasser in Frage.

In einer bevorzugten Ausführungsform der Verfahrensvariante a) wird das Carbonsäurechlorid der Formel II mit einem Amin der Formel III oder dessen Salzen zweckmäßig

in Gegenwart eines säurebindenden Mittels, wie Kalium- oder Natriumcarbonat, Alkali- oder Erdalkalihydroxid oder -alkoholat, einer organischen Base, beispielsweise Triäthylamin, Pyridin, Picolin oder Chinolin oder des im Überschuß eingesetzten Amins der Formel III bei Temperaturen zwischen 0° und 160°C, vorzugsweise zwischen 20° und 80°C, umgesetzt. Die Reaktionszeiten können von wenigen Minuten bis zu zwei Stunden betragen.
Das Produkt der Formel I wird aus dem Trockenrückstand der Reaktionslösung durch Umkristallisieren erhalten. Dazu werden die am Ende des Verfahrens b) genannten organischen vorzugsweise schwach polaren Lösungsmittel verwendet.

Die als Ausgangsstoffe benötigten Isoxazol-4-carbonsäure-Derivate der Formel II werden entsprechend DRP 634 286 durch Umsetzung eines 2-Äthoxymethylenacylessigsäureesters mit Hydroxylamin zu dem entsprechenden Isoxazol-4-carbonsäureester, saures Verseifen des so erhaltenen Esters, z.B. in einem Gemisch aus Eisessig und konzentrierter Salzsäure im Verhältnis 2 : 1, zu der Isoxazol-4-carbonsäure und Überführen dieser Carbonsäure nach üblichen Methoden in die Carbonsäurehalogenide, Ester oder gemischten Anhydride, erhalten.

Zur Durchführung des Verfahrens b) wird ein Acylessigsäureamid der Formel IV mit einem Orthoameisensäureester der Formel V, in zweckmäßig mindestens äquimolarer Menge, vorteilhaft in Gegenwart eines Kondensationsmittels, beispielsweise eines Säureanhydrids, vorzugsweise eines aliphatischen Säureanhydrids mit 4 bis 6 C-Atomen, wie Acetanhydrid, umgesetzt. Das Säureanhydrid kann gleichzeitig als Reaktionsmedium dienen. In diesem Fall wendet man es zweckmäßig in einem 2- bis 4-fach molaren Überschuß an und erwärmt 30 Minuten bis 5 Stunden lang auf eine Temperatur zwischen 80° und 150°C, vorzugsweise auf die Siedetemperatur des jeweiligen Reaktionsgemisches.

Die so erhaltenen 2-Alkoxymethylenacylessigsäureamide der Formel VI sind neu. Sie werden anschließend, gegebenenfalls nach vorheriger Isolierung, mit einer zweckmäßig mindestens äquimolaren Menge Hydroxylamin oder eines Hydroxylammoniumsalzes in einem unter den Reaktionsbedingungen gegenüber den Reaktanten indifferenten Verteilungs- oder Lösungsmittel, gegebenenfalls auch einem Lösungsmittelgemisch, vorzugsweise einem Alkohol, wie Methyl-, Äthyl-, Propyl- oder Isopropylalkohol, eventuell unter Zusatz von bis zu 2 Volumenteilen, vorzugsweise bis zu 1 Volumenteil, Wasser auf 1 Volumenteil organisches Lösungsmittel, bei einer Temperatur zwischen 0° und 130°C, vorzugsweise zwischen 20° und 100°C unter Ringschluß kondensiert. Die Reaktionszeiten können dabei von wenigen Minuten bis zu 5 Stunden betragen.

Bei Anwendung des Hydroxylamins in Form eines Säureadditionssalzes empfiehlt sich der Zusatz von säurebindenden Mitteln, wie Alkali- oder Erdalkalihydroxiden, -carbonaten oder -alkoholaten oder organischen Basen, beispielsweise Diäthyl- oder Triäthylamin.

Das Produkt der Formel I fällt nach Erkalten oder Reaktionsmischung kristallin aus; weitere Mengen werden nach Wasserzusatz erhalten. Das Produkt kann anschließend durch Umkristallisieren gereinigt werden. Dazu verwendet man ein organisches, vorzugsweise mäßig polares Lösungsmittel wie Toluol, ein Dimethylbenzol, Benzol, Cyclohexan, Methanol oder Äthanol, oder ein Gemisch aus solchen Lösungsmitteln.

Die als Ausgangsstoffe benötigten Acylessigsäureamide der Formel IV werden aus den entsprechenden Aminen der Formel III durch Umsetzung mit Diketen in Acetonitril oder Toluol, oder mit Acetessigester erhalten.

Die erfindungsgemäßen Isoxazol-4-carbonsäureamide der Formel I haben antirheumatische, antiphlogistische, analgetische und antipyretische Eigenschaften. Sie sind gut verträglich und können daher als Arzneimittel Verwendung finden. Sie können entweder allein, z.B. in Form von Mikrokapseln, oder vermischt mit geeigneten Trägerstoffen verabreicht werden.

Gegenstand der Erfindung sind somit auch Arzneimittel, die aus einer Verbindung der Formel I bestehen oder diesen Wirkstoff neben einem pharmazeutisch üblichen physiologisch verträglichen Trägerstoff, Verdünnungsmittel und/oder Konstituens enthalten. Die Mittel können oral, rectal oder parenteral appliziert werden, wobei die orale oder rectale Anwendung bevorzugt ist. Geeignete feste oder flüssige galenische Zubereitungsformen sind beispielsweise Granulate, Pulver, Tabletten, Kapseln, Suppositorien, Sirupe, Suspensionen oder Tropfen sowie Präparate mit protrahierter Wirkstofffreigabe. Als häufig verwendete Trägermittel seien z.B. Calciumcarbonat, Calciumphosphate, verschiedene Zukker oder Stärkearten, Cellulosederivate, Gelatine, pflanzliche Öle, Polyäthylenglykole und physiologisch unbedenkliche Lösungsmittel genannt.

Eine weitere Anwendung der Verbindungen gemäß Formel I besteht in deren Kombination mit anderen geeigneten Wirkstoffen, beispielsweise Antiuricopathika, Trombocytenaggregationshemmern, anderen Analgetika und anderen steroidalen oder nichtsteroidalen Antiphlogistika.

Die erfindungsgemäßen Verbindungen der Formel I wurden in den anschließend beschriebenen Tiermodellen vergleichend mit dem bekannten Antiphlogistikum Phenylbutazon und dem N-(4-Fluorphenyl)-5-methylisoxazol-4-carboxamid (Verbindung Nr. 10 aus Tabelle 2 der DE-OS 25 24 959) auf antiphlogistische Wirkung (Adjuvans-Arthritis), ulcerogene Wirkung und akute Toxizität geprüft. Die Ergebnisse

dieser Untersuchungen, die die Überlegenheit der neuen Verbindungen I gegenüber den bekannten Substanzen demonstrieren, sind in der nachfolgenden Tabelle 1 zusammengefaßt.

## Pharmakologische Prüfung und Ergebnisse

### 1. Adjuvans-Arthritis, praeventive Testung

Die Untersuchungen wurden nach der Methode von Pearson durchgeführt (Arthrit. Rheum. 2, 440 (1959)). Als Versuchstiere dienten männliche Ratten eines Wistar-Lewis-Stammes im Körpergewicht zwischen 130 und 200 g. Die zu prüfenden Verbindungen wurden täglich vom 1. bis 17. Versuchstag oral appliziert. Tiere einer Kontrollgruppe erhielten nur das Lösungsmittel. Als Wirkungskriterium diente die Herabsetzung der Pfoten-Volumenzunahme gegenüber der unbehandelten Kontrollgruppe. Die $ED_{50}$-Werte wurden graphisch aus der Dosiswirkungskurve bestimmt.

### 2. Ulzerogene Wirkung

Die Untersuchungen erfolgten an männlichen Sprague-Dawley Ratten im Körpergewicht zwischen 200 und 300 g. 48 Stunden vor Applikation der Testsubstanzen bis zum Töten der Tiere wurde das Futter entzogen, bei freiem Zugang zum Trinkwasser. Die Ratten wurden 24 Stunden nach oraler Gabe getötet, der Magen entommen, unter fließendem Wasser gereinigt und auf Schleimhautlösionen inspiziert. Als Ulzera galten alle makroskopisch sichtbaren Läsionen. Bestimmt wurde der Anteil der Tiere mit Ulzera pro dosi und die $UD_{50}$ nach Litchfield und Wilcoxon (J. Pharmacol. exp. Ther. 96, 99 (1949)).

3. Akute Toxizität

Die Bestimmung der $LD_{50}$-Bereiche erfolgte standardgemäß über die innerhalb von 7 Tagen bei NMRI-Mäusen nach intraperitonealer Gabe auftretende Mortalität.

Tabelle 1

| Verbindung des Beispiels | Adjuvans-Arthritis $ED_{50}$ (mg/kg) | Ulzerogenität $UD_{50}$ (mg/kg) | Therapeut. Index $\frac{UD_{50}}{ED_{50}}$ | Toxizität $LD_{50}$-Bereich (Maus i.p.) in mg/kg |
|---|---|---|---|---|
| 1 | 6 | 70 | 12 | 150 - 300 |
| 6 | 3 | 80 | 27 | |
| 10 | 8 | 230 | 29 | 150 - 300 |
| P 25 24 959 Nr. 10 | 15 | 250 | 17 | 200 - 400 |
| Phenyl=butazon | 37 | 100 | 3 | $LD_{50}$ = 336* |

* C.D. Barnes und L. G. Eltherington; Drug Dosage in Laboratory Animals, Verlag: University of California Press-Berkeley Los Angeles, 1973, Seite 199.

Die Arzneimittel gemäß der Erfindung enthalten den Wirkstoff der Formel I zur oralen Applikation, z.B. in Form von Kapseln, in Dosen von 25 - 250 mg, vorzugsweise 50 - 200 mg, zur rektalen Applikation, z.B. in Form von Suppositorien, in Dosen von 50 - 500 mg, vorzugsweise 100 - 400 mg. Diese Arzneimittel sind je nach Lage des Falles ein bis viermal, im Mittel zwei- bis dreimal täglich zu applizieren. Für Suspensionen oder

Lösungen kommt ein Gehalt an Wirkstoff der Formel I von 0,1 bis 20 Gew.-% vorzugsweise 1 - 10 Gew.-% in Betracht.

Herstellungsbeispiele

1. N-(4-Trifluormethylphenyl)-5-äthylisoxazol-4-carboxamid
Verfahren a)

$a_1$) 0,1 Mol (16,1 g) 4-Trifluormethylanilin, gelöst in 150 ml Acetonitril, werden bei Raumtemperatur tropfenweise mit einer Lösung von 0,05 Mol (8,0 g) 5-Äthylisoxazol-4-carbonsäurechlorid in 20 ml Acetonitril unter Rühren versetzt. Man rührt weitere 20 Minuten und filtriert die Flüssigkeit vom ausgefallenen Salz ab. Das Filtrat wird unter vermindertem Druck zur Trockene gebracht. Man erhält so 13,1 g (92,1 % d.Th.) kristallines Produkt. Schmelzpunkt (aus Toluol): 104° - 105°C.

$a_2$) 0,1 Mol (16,1 g) 4-Trifluormethylanilin und 0,1 Mol (28,6 g) (2,4-Dichlor)phenyl-5-äthylisoxazol-4-carboxylat, gelöst in 180 ml Tetrahydrofuran, werden 2 Stunden unter Rückfluß erhitzt. Man verdampft das Lösungsmittel unter vermindertem Druck und gewinnt aus dem Rückstand durch Umkristallisieren aus Toluol N-(4-Trifluormethylphenyl)-5-äthylisoxazol-4-carboxamid vom Schmelzpunkt 104° - 105°C.

b) Dieselbe Verbindung erhält man durch Umsetzung von N-(4-Trifluormethylphenyl)-2-äthoxymethylenpropionylacetamid mit Hydroxylamin nach Verfahren b).

2. N-/4-(4-Trifluormethylphenoxy)-phenyl/-5-äthylisoxazol-4-carboxamid

$a_1$) Zu einer Lösung von 0,1 Mol (29,2 g) 4-(4-Trifluormethylphenoxy)-anilinhydrochlorid und 0,1 Mol (10,1 g) Triäthylamin in 200 ml Acetonitril gibt man bei Raumtemperatur unter Rühren tropfenweise 0,05 Mol (8,0 g) 5-Äthylisoxazol-4-carbonsäurechlorid, gelöst in 20 ml Acetonitril, hinzu. Nach 10 Minuten Rühren filtriert man die Flüssigkeit von den ausgefallenen Salzen ab. Das Filtrat wird unter vermindertem Druck zur Trockene gebracht. Der Rückstand wird mit je 300 ml Methylenchlorid und 1 n Salzsäure aufgenommen und extrahiert. Die Methylenchloridphase wird mit Wasser neutral gewaschen, getrocknet und im Rotationsverdampfer unter vermindertem Druck zur Trockne gebracht. Man erhält so 15,9 g (84,5 % d.Th.) kristallines Produkt vom Schmelzpunkt (aus Toluol) 92° - 94°C.

a $_3$) 0,1 Mol (29,0 g) 4-(4-Trifluormethylphenoxy)-anilin-hydrochlorid, 0,1 Mol (10,1 g) Triäthylamin und 0,1 Mol (21,3 g) Äthoxycarbonyl-5-äthylisoxazol-4-carboxylat, gelöst in 200 ml Dioxan, werden 1,5 Stunden unter Rückfluß erhitzt. Danach filtriert man die Flüssigkeit von den ausgefallenen Salzen ab. Das Filtrat wird unter vermindertem Druck zur Trockne gebracht. Der Rückstand wird mit je 300 ml Methylenchlorid und 1 n Salzsäure aufgenommen und extrahiert. Die Methylenchloridphase wird mit Wasser neutral gewaschen, getrocknet und unter vermindertem Druck zur Trockne gebracht. Aus dem Rückstand gewinnt man durch Umkristalisieren aus Toluol N-/4-(4-Trifluormethylphenoxy)-phenyl7-5-äthylisoxazol-4-carboxamid (Schmp. 92° - 94°C ).

a $_3$) 0,1 Mol (29,0 g) 4-(4-Trifluormethylphenoxy)-anilin-hydrochlorid, 0,1 Mol (10,1 g) Triäthylamin und 0,1 Mol (27,5 g) Benzyloxycarbonyl-5-äthylisoxazol-4-carboxylat, gelöst in 200 ml Acetonitril, werden 2 Stunden unter Rückfluß erhitzt. Nach Abkühlen auf Raumtemperatur filtriert man die Flüssigkeit von den ausgefallenen Salzen ab. Das Filtrat wird

unter vermindertem Druck zur Trockne gebracht. Der Rückstand wird mit je 300 ml Methylenchlorid und 1 n Salzsäure aufgenommen und extrahiert. Die Methylenchloridphase wird mit Wasser neutral gewaschen, getrocknet und unter vermindertem Druck zur Trockne gebracht. Aus dem Rückstand isoliert man durch Umkristallisieren aus oluol N-/4-(4-Trifluormethylphenoxy)-phenyl7-5-äthylisoxazol-4-carboxamid (Schmp. 92° - 94°).

b) Dieselbe Verbindung erhält man durch Umsetzung von N-/4-(4-Trifluormethylphenyloxy)-phenyl7-2-äthoxymethylenpropionylacetamid mit Hydroxylamin nach Verfahren b).

3. N-Methyl-N-(4-trifluormethylphenyl)-5-methylisoxazol-4-carboxamid - Verfahren b)

Stufe 1:

0,1 Mol (25,9 g) N-Methyl-N-(4-trifluormethylphenyl)-acetoacetamid werden mit 0,11 Mol (16,3 g) Orthoameisensäuretriäthylester und 0,3 Mol (30,6 g) Acetanhydrid 2 Stunden unter Rückfluß gekocht. Nach dem Abkühlen auf Zimmertemperatur wird die Flüssigkeit von den ausgefallenen Kristallen abgesaugt und die Kristalle mit wenig Acetanhydrid und dann mit Petroläther gewaschen. Man erhält so 20,2 g (64,1 % der Theorie) N-Methyl-(4-trifluormethylphenyl)-2-äthoxymethylenacetoacetamid, das ohne weitere Reinigung in Stufe 2 eingesetzt werden kann.

Stufe 2:

0,11 Mol (7,65 g) Hydroxylammoniumchlorid werden in 50 ml Wasser gelöst und mit einer eiskalten Lösung von 0,11 Mol (4,4 g) Natriumhydroxid in 10 ml Wasser versetzt. Anschließend läßt man in diese Hydroxylaminlösung bei 5° bis 10°C 0,1 Mol (31,5 g) der Ver-

bindung aus Stufe 1, gelöst in 60 ml Äthanol, zutropfen. Man erhitzt dann 30 Minuten unter Rückfluß. Die nach dem Abkühlen ausgefallenen Kristalle werden abfiltriert mit Wasser gewaschen und getrocknet. Man erhält so 22,3 g (78,5 % der Theorie) kristallines N-Methyl-N-(4-trifluormethylphenyl)-5-methylisoxazol-4-carboxamid vom Schmelzpunkt. 105° - 107°C (aus Methanol). Weitere Produktmengen werden nach Zusatz von Wasser zur Reaktionsmischung erhalten.

a) Dieselbe Verbindung erhält man durch Umsetzung von N-Methyl-4-trifluormethylanilin mit 5-Methylisoxazol-4-carbonsäurechlorid entsprechend Verfahren a).

Nach den Verfahren a) oder b) analog den vorstehend beschriebenen Beispielen werden die folgenden Verbindungen der Formel I dargestellt.

4. <u>N-(4-Trifluormethylphenyl)-5-propylisoxazol-4-carboxamid</u> (vom Schmelzpunkt 123° bis 130°C)

a) aus 5-Propylisoxazol-4-carbonsäurechlorid und 4-Trifluormethylanilin bzw.
b) aus N-(4-Trifluormethylphenyl)-2-äthoxymethylenbutyrylacetamid und Hydroxylamin.

5. <u>N-(4-Trifluormethylphenyl)-5-butylisoxazol-4-carboxamid</u> (vom Schmelzpunkt 105 ° bis 106 ° C) aus 5-Butylisoxazol-4-carbonsäurechlorid und 4-Trifluor=methylanilin bzw. aus N-(4-Trifluormethylphenyl)-2-äthoxymethylenvalerylacetamid und Hydroxylamin.

6. <u>N-(4-Trifluormethylphenyl)-5-trifluormethylisoxazol-4-carboxamid</u> (vom Schmelzpunkt 128 ° bis 130 ° C) aus 5-Trifluormethylisoxazol-4-carbonsäurechlorid und 4-Trifluormethylanilin bzw. aus N-(4-Trifluormethylphenyl)-2-äthoxymethylentrifluoracetylacetamid und Hydroxylamin.

7. N-(3-Chlor-4-trifluormethylphenyl)-5-methylisoxazol-4-carboxamid (vom Schmelzpunkt 139 ° bis 144 ° C) aus 5-Methylisoxazol-4-carbonsäurechlorid und 3-Chlor-4-trifluormethylanilin bzw. aus N-(3-Chlor-4-trifluor=methylphenyl)-2-äthoxymethylenacetoacetamid und Hydroxyl=amin.

8. N-(2-Chlor-4-trifluormethylphenyl)-5-methylisoxazol-4-carboxamid (vom Schmelzpunkt 106 ° bis 107 ° C) aus 5-Methylisoxazol-4-carbonsäurechlorid und 2-Chlor-4-trifluormethylanilin bzw. aus N-(2-Chlor-4-trifluormethyl=phenyl)-2-äthoxymethylenacetoacetamid und Hydroxylamin.

9. N-(3-Chlor-4-trifluormethylphenyl)-5-äthylisoxazol-4-carboxamid (vom Schmelzpunkt 157 ° bis 160 ° C) aus 5-Äthylisoxazol-4-carbonsäurechlorid und 3-Chlor-4-trifluormethylanilin bzw. aus N-(3-Chlor-4-trifluormethyl=phenyl)-2-äthoxymethylenpropionylacetamid und Hydroxylamin.

10. N-[4-(4-Trifluormethylphenoxy)-phenyl]-5-methylisoxazol-4-carboxamid (vom Schmelzpunkt 115 ° bis 117 ° C) aus 5-Methylisoxazol-4-carbonsäurechlorid und 4-(4-Trifluormethylphenoxy)anilin bzw. aus N-[4-(4-Trifluor=methylphenoxy)-phenyl]-2-äthoxymethylenacetoacetamid und Hydroxylamin.

11. N-[4-(3-Chlor-4-trifluormethylphenoxy)-phenyl]-5-methyl-isoxazol-4-carboxamid (vom Schmelzpunkt 175 ° bis 180 ° C) aus 5-Methylisoxazol-4-carbonsäurechlorid und 4-(3-Chlor-4-trifluormethylphenoxy)anilin bzw. aus N-[4-(3-Chlor-4-trifluormethylphenoxy)-phenyl]-2-äthoxymethylenacetoacet=amid und Hydroxylamin.

12. N-[4-(4-Trifluormethylbenzoyl)-phenyl]-5-methylisoxazol-4-carboxamid aus 5-Methylisoxazol-4-carbonsäurechlorid und 4-(4-Trifluormethylbenzoyl)anilin.

13. N-[4-(4-Trifluormethylbenzoyl)-phenyl]-5-äthylisoxazol-4-carboxamid aus 5-Äthylisoxazol-4-carbonsäurechlorid und 4-(4-Trifluormethylbenzoyl)anilin.

14. N-[4-(4-Trifluormethylbenzoyl)-phenyl]-5-trifluormethyl=isoxazol-4-carboxamid aus 5-Trifluormethylisoxazol-4-carbonsäurechlorid und 4-(4-Trifluormethylbenzoyl)anilin.

15. N-[4-(4-Trifluormethylbenzoyl)-phenyl]-isoxazol-4-carboxamid aus Isoxazol-4-carbonsäurechlorid und 4-(4-Tri=fluormethylbenzoyl)anilin.

16. N-[4-(4-Trifluormethylphenoxy)-phenyl]-5-trifluormethyl=isoxazol-4-carboxamid vom Schmp. 107-108° aus 5-Trifluormethylisoxazol-4-carbonsäurechlorid und 4-(4-Trifluormethylphenoxy)anilin bzw. aus N-[4-(4-Trifluormethylphenoxy)-phenyl]-2-äthoxy=methylentrifluoracetylacetamid und Hydroxylamin.

17. N-(5-Trifluormethyl-2-pyridyl)-5-trifluormethylisoxazol-4-carboxamid aus 5-Trifluormethylisoxazol-4-carbonsäure=chlorid und 2-Amino-5-trifluormethylpyridin.

18. N-(5-Trifluormethyl-2-pyridyl)-5-methylisoxazol-4-carboxamid aus 5-Methylisoxazol-4-carbonsäurechlorid und 2-Amino-5-trifluormethylpyridin.

19. N-Methyl-N-(5-trifluormethyl-2-pyridyl)-5-äthylisoxazol-4-carboxamid aus 5-Äthylisoxazol-4-carbonsäurechlorid und 2-Methylamino-5-trifluormethylpyridin bzw. aus N-Methyl-N-(5-trifluormethyl-2-pyridyl)-2-äthoxymethylenpropionyl=acetamid und Hydroxylamin.

20. N-(5-Trifluormethyl-2-pyridyl)-5-äthylisoxazol-4-carboxamid aus 5-Äthylisoxazol-4-carbonsäurechlorid und 2-Amino-5-trifluormethylpyridin.

21. N-(5-Trifluormethyl-2-pyridyl)-isoxazol-4-carboxamid aus Isoxazol-4-carbonsäurechlorid und 2-Amino-5-trifluor=methylpyridin.

22. N-(4-Trifluormethylphenyl)-isoxazol-4-carboxamid vom Schmp. 174-180°C aus Isoxazol-4-carbonsäurechlorid und 4-Trifluormethylanilin.

23. N-/4-(4-Trifluormethylphenoxy)-phenyl7-isoxazol-4-carboxamid vom Schmp. 158 - 160°C aus Isoxazol-4-carbonsäurechlorid und 4-(4-Trifluormethylphenoxy)anilin.

Die vorgenannten Verbindungen sind mit ihren Strukturelementen in der Tabelle 2 zusammengefaßt.

<u>Tabelle 2:</u> Verbindungen der Formel I

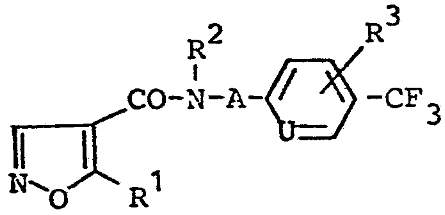

| Beispiel-Nr. | $R^1$ | $R^2$ | A | U | $R^3$ |
|---|---|---|---|---|---|
| 1 | $-C_2H_5$ | H | - | CH | H |
| 2 | $-C_2H_5$ | H | 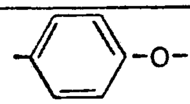 | CH | H |
| 3 | $-CH_3$ | $-CH_3$ | - | CH | H |
| 4 | $-C_3H_7$ | H | - | CH | H |
| 5 | $-C_4H_9$ | H | - | CH | H |
| 6 | $-CF_3$ | H | - | CH | H |
| 7 | $-CH_3$ | H | - | CH | 3-Cl |
| 8 | $-CH_3$ | H | - | CH | 2-Cl |
| 9 | $-C_2H_5$ | H | - | CH | 3-Cl |
| 10 | $-CH_3$ | H | 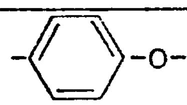 | CH | H |
| 11 | $-CH_3$ | H | 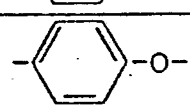 | CH | 3-Cl |

Tabelle 2 (Fortsetzung)

| Beispiel-Nr. | $R^1$ | $R^2$ | A | U | $R^3$ |
|---|---|---|---|---|---|
| 12 | $-CH_3$ | H | 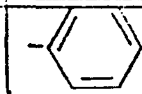-CO- | CH | H |
| 13 | $-C_2H_5$ | H | 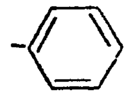-CO- | CH | H |
| 14 | $-CF_3$ | H | 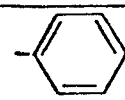-CO- | CH | H |
| 15 | H | H | 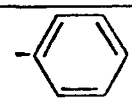-CO- | CH | H |
| 16 | $-CF_3$ | H | 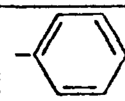-O- | CH | H |
| 17 | $-CF_3$ | H | - | N | H |
| 18 | $-CH_3$ | H | - | N | H |
| 19 | $-C_2H_5$ | $-CH_3$ | - | N | H |
| 20 | $-C_2H_5$ | H | - | N | H |
| 21 | H | H | - | N | H |
| 22 | H | H | - | CH | H |
| 23 | H | H | 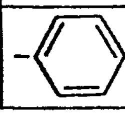-O- | CH | H |

Herstellung der Ausgangsstoffe

Die nachstehenden Isoxazol-4-carbonsäurechloride der Formel II werden durch Umsetzung der entsprechenden Isoxazol-4-carbonsäuren mit Phosphorpentachlorid in Tetrachlorkohlenstoff oder Cyclohexan erhalten:

| $R^1$ | Kp.: /°C/mm Hg/ |
|---|---|
| H | 63 - 70 / 12 |
| $CH_3$ | 71 - 72 / 9 |
| $C_2H_5$ | 78 - 79 / 9 |
| n-$C_3H_7$ | 105 -110 / 12 |
| n-$C_4H_9$ | 107 -109 / 15 |

Das Isoxazol-4-carbonsäurechlorid der Formel II mit $R^1$ = $CF_3$ wurde ohne Destillation weiter verarbeitet.

Isoxazol-4-carbonsäure ist bekannt nach Gazz. Chim. Ital. 96 (4) 443-453 (1966).

Die entsprechenden Isoxazol-4-carbonsäuren werden analog DRP 634 286 durch Umsetzen der entsprechenden 2-Äthoxyalkylidenacetessigester mit Hydroxylamin und saures Verseifen der erhaltenen Isoxazol-4-carbonsäureester dargestellt:

| $R^1$ | Schmp. /°C / Lösungsm./ |
|---|---|
| $CH_3$ | 147 - 148 / Cyclohexan |
| $C_2H_5$ | 85 - 87 / Cyclohexan |
| n-$C_3H_7$ | 88 - 91 / Cyclohexan |
| n-$C_4H_9$ | 54 - 57 / Petroläther |
| $CF_3$ | 75 - 78 / Toluol |

Amine der Formel III:

Zur Herstellung von 4-(4-Trifluormethylphenoxy)anilin werden p-Acetamidophenol, 4-Chlorbenzotrifluorid und $K_2CO_3$ in Dimethylsulfoxyd 22 Stunden unter Rückfluß erhitzt. Das nach Aufarbeitung erhaltene N-/4-(4-Trilfuormethylphenoxy)7acetanilid (Schmp. 142-144°C) wird mit 20%iger Salzsäure zum 4-(4-Trifluormethylphenoxy)anilin-Hydrochlorid verseift und aus Wasser umkristallisiert Schmp. 181 - 194°C (Zers.).

N-Methyl-4-trifluormethylanilin wird analog Roberts und Vogt, J. Am. Chem. Soc., 78, 4478 (1956) aus 4-Trifluormethylanilin und Orthoameisensäuretrimethylester und anschließende Verseifung des N-Methyl-4-trifluormethylformanilids und Reinigung durch Destillation hergestellt (Siedebereich 58 - 72°C / 12 mm Hg).

3-Chlor-4-trifluormethylanilin wird durch alkalische Verseifung des 3-Chlor-4-trifluormethylphenylisocyanats gewonnen und ohne weitere Reinigung mit den entsprechenden Isoxazol-4-carbonsäurechloriden der Formel II umgesetzt.

3-Chlor-trifluormethylphenylisocyanat ist bekannt aus der deutschen Patentschrift 16 68 003.

2-Chlor-4-trifluormethylanilin läßt sich analog 3-Chlor-4-trifluormethylanilin aus 2-Chlor-4-methylanilin über das 2-Chlor-4-methylphenylisocyanat, dessen Chlorierung zum 2-Chlor-4-trichlormethylphenylisocyanat und Überführen mit Antimontrifluorid in das 2-Chlor-4-trifluormethylphenylisocyanat durch alkalische Verseifung darstellen; (Siedepunkt 99 - 101°C / 15 mm Hg).

4-(3-Chlor-4-trifluormethyl)phenoxyanilin-Hydrochlorid wurde analog 4-(4-Trifluormethylphenoxy)anilin aus p-Acetamidophenol und 2,4-Dichlorbenzotrifluorid erhalten;

Schmp. 154-158°C (aus Wasser).

Patentansprüche:

1. Isoxazol-4-carbonsäureamide der allgemeinen Formel I

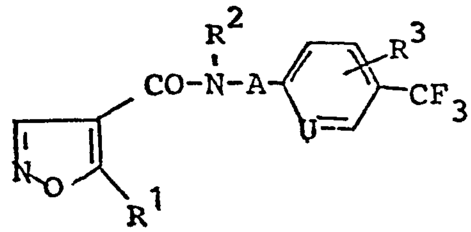
(I),

in der
$R^1$ Wasserstoff, Alkyl oder Halogenalkyl mit jeweils bis zu 4 C-Atomen,
$R^2$ Wasserstoff oder $(C_1-C_4)$Alkyl,
$R^3$ Wasserstoff oder Halogen,
U die -CH-Gruppe oder Stickstoff und
A den Rest der Formel

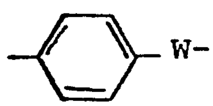

in der W für Sauerstoff oder die Carbonylgruppe steht, oder eine Einfachbindung, in welchem Fall jedoch $R^1$ dann verschieden von Methyl ist, wenn gleichzeitig $R^2$ und $R^3$ Wasserstoff und U die CH-Gruppe darstellen, bedeuten.

2. Verfahren zur Herstellung von Verbindungen der Formel I, dadurch gekennzeichnet, daß man

a) ein Isoxazol-4-carbonsäure-Derivat der Formel II

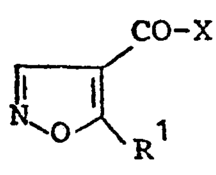
(II)

in der X entweder ein Halogenatom, vorzugsweise Chlor oder Brom, eine YO- oder ZO-CO-O-Gruppe bedeutet, wobei Y für gegebenenfalls durch Fluor, Chlor, Brom, Jod, Methyl, Äthyl, Methoxy, Äthoxy, Trifluormethyl, Nitro oder Cyan einfach, zweifach oder dreifach substituiertes Phenyl oder für den Acylrest entsprechend der Formel II (Formel II ohne X) und Z für $(C_1-C_4)$ Alkyl, Phenyl oder Benzyl stehen, und $R^1$ Wasserstoff, Alkyl oder Halogenalkyl mit jeweils bis zu 4 C-Atomen bedeutet, mit einem Amin der Formel III oder dessen Salzen,

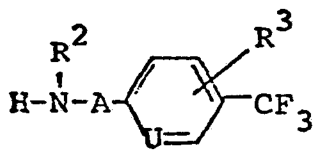

(III),

in der $R^2$, $R^3$, U und A die vorgenannten Bedeutungen haben, umsetzt, oder

b) ein Acylessigsäureamid der Formel IV

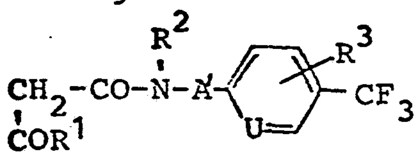

(IV),

in der

$R^1$ Alkyl oder Halogenalkyl mit jeweils bis zu 4 C-Atomen,

$R^2$ Wasserstoff oder $(C_1-C_4)$-Alkyl,

$R^3$ Wasserstoff oder Halogen,

U die CH-Gruppe oder Stickstoff und

A' den Rest der Formel 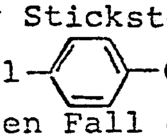 -O- oder eine Einfachbindung, in welchem letzteren Fall einerseits $R^1$ dann verschieden von Methyl ist, wenn gleichzeitig $R^2$ und $R^3$ Wasserstoff und U die CH-Gruppe darstellen, oder andererseits nicht gleichzeitig $R^2$ Wasserstoff und U Stickstoff sind, bedeuten,

mit einem Orthoameisensäureester der Formel V

$HC(OR)_3$ (V),

worin R eine $(C_1-C_4)$-Alkylgruppe, vorzugsweise Methyl oder Äthyl, darstellt, zu 2-Alkoxymethylenacylessigsäureamid der Formel VI

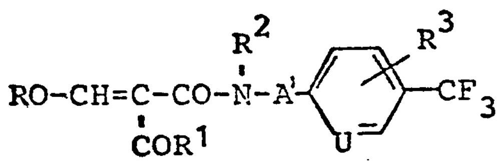

(VI)

in der die Substituenten die vorstehend angegebenen Bedeutungen haben, umsetzt und dieses anschließend mit Hydroxylamin unter Ringschluß kondensiert.

3. Arzneimittel, gekennzeichnet durch den Gehalt an einer Verbindung der Formel I nach Anspruch 1, in Mischung mit einem physiologisch verträglichen Trägerstoff und/oder Konstituens.

4. Verwendung der Verbindungen nach Anspruch 1 zur Behandlung von Entzündungen oder rheumatischen Beschwerden.

5. N-(4-Trifluormethylphenyl)-5-äthylisoxazol-4-carboxamid

6. N-(4-Trifluormethylphenyl)-5-trifluormethylisoxazol-4-carboxamid

7. N-/(4-Trifluormethylphenoxy)-phenyl/-5-methylisoxazol-4-carboxamid

8. 2-Alkoxymethylenacylessigsäureamide der Formel VI

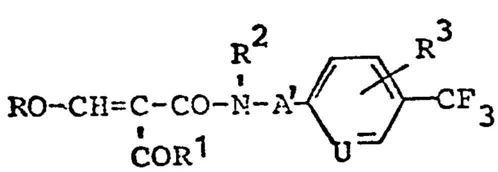

(VI)

in der

R $(C_1-C_4)$Alkyl

$R^1$ Alkyl oder Halogenalkyl mit jeweils bis zu 4 C-Atomen,

$R^2$ Wasserstoff oder $(C_1-C_4)$-Alkyl,

$R^3$ Wasserstoff oder Halogen,

U die CH-Gruppe oder Stickstoff und

A' den Rest der Formel 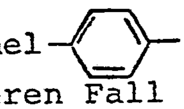 O- oder eine Einfachbindung, in welchem letzteren Fall einerseits $R^1$ dann verschieden von Methyl ist, wenn gleichzeitig $R^2$ und $R^3$ Wasserstoff und U die CH-Gruppe darstellen, oder andererseits nicht gleichzeitig $R^2$ Wasserstoff und U Stickstoff sind, bedeuten.

**Patentanspruch für Österreich:**

1. Verfahren zur Herstellung von Isoxazol-4-carbonsäureamiden der allgemeinen Formel I

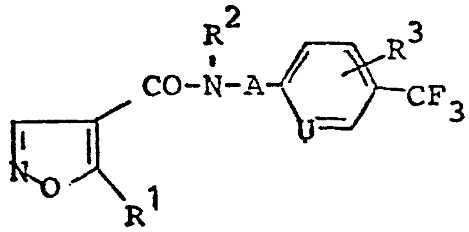

(I),

in der

$R^1$ Wasserstoff, Alkyl oder Halogenalkyl mit jeweils bis zu 4 C-Atomen,

$R^2$ Wasserstoff oder $(C_1-C_4)$Alkyl,

$R^3$ Wasserstoff oder Halogen,

U die -CH-Gruppe oder Stickstoff und

A den Rest der Formel

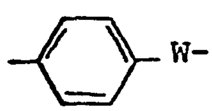

in der W für Sauerstoff oder die Carbonylgruppe steht, oder eine Einfachbindung, in welchem Fall jedoch $R^1$ dann verschieden von Methyl ist, wenn gleichzeitig $R^2$ und $R^3$ Wasserstoff und U die CH-Gruppe darstellen, bedeuten, dadurch gekennzeichnet, daß man

a) ein Isoxazol-4-carbonsäure-Derivat der Formel II

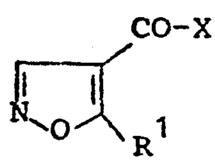

(II)

in der X entweder ein Halogenatom, vorzugsweise Chlor oder Brom, eine YO- oder ZO-CO-O-Gruppe bedeutet, wobei Y für gegebenenfalls durch Fluor, Chlor, Brom, Jod, Methyl, Äthyl, Methoxy, Äthoxy, Trifluormethyl, Nitro oder Cyan einfach, zweifach oder dreifach substituiertes Phenyl oder für den Acylrest entsprechend der Formel II (Formel II ohne X) und Z für $(C_1-C_4)$ Alkyl, Phenyl oder Benzyl stehen, und $R^1$ Wasserstoff, Alkyl oder Halogenalkyl mit jeweils bis zu 4 C-Atomen bedeutet, mit einem Amin der Formel III oder dessen Salzen,

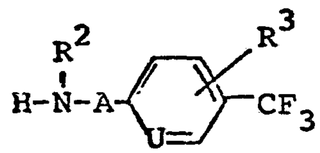

(III),

in der $R^2$, $R^3$, U und A die vorgenannten Bedeutungen haben, umsetzt oder

b) ein Acylessigsäureamid der Formel IV

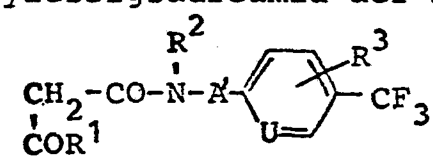

(IV),

in der

$R^1$ Alkyl oder Halogenalkyl mit jeweils bis zu 4 C-Atomen,

$R^2$ Wasserstoff oder $(C_1-C_4)$-Alkyl,

$R^3$ Wasserstoff oder Halogen,

U die CH-Gruppe oder Stickstoff und

A' den Rest der Formel 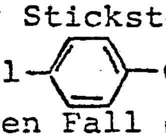 -O- oder eine Einfachbindung, in welchem letzteren Fall einerseits $R^1$ dann verschieden von Methyl ist, wenn gleichzeitig $R^2$ und $R^3$ Wasserstoff und U die CH-Gruppe darstellen, oder andererseits nicht gleichzeitig $R^2$ Wasserstoff und U Stickstoff sind, bedeuten,

mit einem Orthoameisensäureester der Formel V

$$HC(OR)_3 \qquad (V),$$

worin R eine $(C_1-C_4)$-Alkylgruppe, vorzugsweise Methyl oder Äthyl, darstellt,
zu 2-Alkoxymethylenacylessigsäureamid der Formel VI

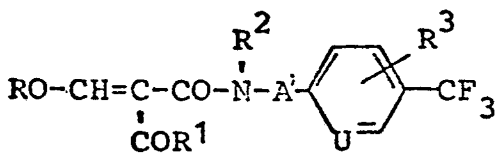

(VI)

in der die Substituenten die vorstehend angegebenen Bedeutungen haben, umsetzt und dieses anschließend mit Hydroxylamin unter Ringschluß kondensiert.